(19) Europäisches Patentamt
European Patent Office
Office européen des brevets

(11) Veröffentlichungsnummer: **0 231 890**
**A2**

(12) # EUROPÄISCHE PATENTANMELDUNG

(21) Anmeldenummer: **87101253.0**

(22) Anmeldetag: **29.01.87**

(51) Int. Cl.$^3$: **C 07 H 15/04**

(30) Priorität: **06.02.86 DE 3603581**

(43) Veröffentlichungstag der Anmeldung:
**12.08.87 Patentblatt 87/33**

(84) Benannte Vertragsstaaten:
**AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Schmid, Karl Heinz, Dr.**
**Stifterstrasse 10**
**D-4020 Mettmann(DE)**

(72) Erfinder: **Biermann, Manfred, Dr.**
**Marktscheiderhof 25**
**D-4330 Mülheim/Ruhr(DE)**

(54) **Verfahren zur Reinigung von Alkylglycosiden, durch dieses Verfahren erhältliche Produkte und deren Verwendung.**

(57) Zur destillativen Trennung von Gemischen aus Alkylglycosiden und Fettalkoholen setzt man den Gemischen ein oder mehrere viskositätssenkende Mittel, die thermostabil und farbstabil im Temperaturbereich bis 160 °C sind und mit den Alkylglycosiden kompatibel sind, und die bei 760 mbar einen Siedepunkt aufweisen, der wenigstens 50 °C über dem Siedepunkt des in der Reaktionsmischung enthaltenden Fettalkohols liegt zu, wobei man eine Viskosität des Gemischs im Temperaturbereich bis 160 °C von höchstens 10 000 mPas einstellt. Aus diesem Gemisch trennt man die Fettalkohole in an sich bekannten Vorrichtungen zur Destillation bei Temperaturen von mindestens 20 °C unterhalb des thermischen Zersetzungspunktes der Alkylglycoside und bei Drücken von 1 bis $10^{-3}$ bar durch Destillation quantitativ ab. Die Verfahrensprodukte enthalten Alkylglycoside in einer Menge von 90 bis 50 Gew.-% und die genannten viskositätssenkenden Zusätze in einer Menge von 10 – 50. Gew.-%. Man verwendet die Verfahrensprodukte zur Herrstellung von Kosmetika, von Wasch-, Spül- und Reinigungsmittel für Haushalts- und Industriezwecke.

EP 0 231 890 A2

0231890



Patentanmeldung D7038 EP

HENKEL KGaA
ZR-FE/Patente
Dr. Ms/Ne
03.02.1986

# Verfahren zur Reinigung von Alkylglycosiden, durch dieses Verfahren erhältliche Produkte und deren Verwendung

Die Erfindung betrifft ein Verfahren zur Reinigung von Alkylglycosiden durch Destillation, die als Produkte eines derartigen Destillationsprozesses erhaltenen Zusammensetzungen sowie deren Verwendung.

Alkylglycoside entstehen durch Umsetzung von Monosacchariden mit Fettalkoholen. Die entstehenden Produkte lassen sich vorteilhaft für eine Vielzahl von Zwecken verwenden, beispielsweise als Detergentien, Netzmittel, Geliermittel, Schmiermittel, Nahrungsmittelemulgatoren oder ähnliches.

Die Herstellung von Alkylmonoglycosiden nach dem Fischer-Verfahren, bei dem ein Monosaccharid und ein Fettalkohol mit einer Zahl von bis zu 10 C-Atomen im Alkylrest in Gegenwart eines sauren Katalysators miteinander umgesetzt werden, ist seit langer Zeit bekannt. Alkylglycoside, in denen die Alkylgruppe bis zu 10 C-Atome aufweist, werden verbreitet als Tenside verwendet. Die Reaktion beider Komponenten miteinander

läuft mit hoher Selektivität ab, und die Hydrophilie der erhaltenen Alkylglycoside läßt sich durch das Molverhältnis Fettalkohol : Monosaccharid einstellen. Dabei weisen Produkte mit 2 oder mehr Monosaccharid-Einheiten eine höhere Wasserlöslichkeit auf als Alkylmonoglycoside.

Verfahren zur Herstellung von Alkylmonoglycosiden bzw. Alkyloligoglycosiden und deren Gemischen, in denen die Alkylgruppe 8 bis 25 C-Atomen enthält, sind ebenfalls grundsätzlich aus der DE-PS 19 05 523, der DE-OS 20 36 472 und der US-PS 3 839 318 bekannt. Die in diesen Druckschriften beschriebenen Herstellungsverfahren weisen jedoch mehrere, für technische Synthesen bedeutsame Nachteile auf. So sind Monosaccharide und Fettalkohole nur sehr begrenzt miteinander mischbar, was die zwingende Gegenwart eines geeigneten, mit beiden Komponenten kompatiblen Lösungsmittels erfordert, das in einer nachfolgenden Prozeßstufe abgetrennt und in das Verfahren zurückgeführt werden muß. Außerdem bilden sich bei der Reaktion in oft unerwünschtem Ausmaß Oligomere und farbgebende Vebindungen, die später durch aufwendige Verfahren abgetrennt werden müssen. Außerdem läßt sich die Hydrophilie der erhaltenen Alkylglycoside durch die Zahl der Monosaccharid-Einheiten nicht in gewünschter Weise steuern.

Der Hauptnachteil der Verfahren nach dem Stand der Technik ist jedoch darin zu sehen, daß stets nur Alkylglycosid-Fettalkohol-Gemische erhalten werden, in denen das Verhältnis Alkylglycosid : Fettalkohol im Bereich von 1 : 10 bis 2 : 1 liegt. Vor einer anwendungstechnischen Nutzung der Alkylglycoside ist also ihre Reinigung von nicht umgesetztem Fettalkohol unvermeidlich.

In der US-PS 3 839 318 ist zwar offenbart, daß der überschüssige Fettalkohol mit Hilfe eines Lösungsmittels im Extraktionsverfahren aus dem erhaltenen Gemisch abgetrennt werden kann. Diese Methode ist jedoch technisch sehr aufwendig, da sie den Einsatz eines zusätzlichen Lösungsmittels erfordert, das in einer weiteren Prozeßstufe abgetrennt und aus Gründen der Materialersparnis zurückgewonnen und wieder in den Prozeß eingeschleust werden muß. Der Prozeß ist damit nicht einstufig durchführbar.

Demgegenüber ist die Reinigung der erhaltenen Alkylglycoside durch Abdestillation des überschüssigen Fettalkohols vorzuziehen. Dabei ergeben sich jedoch technische Schwierigkeiten, da die Alkylglycoside Schmelzpunkte oberhalb von 70°C aufweisen. Mit zunehmendem Glycosidierungsgrad der Alkylglycoside steigt der Schmelzpunkt sogar noch an. Im Bereich der Schmelztemperatur ist die Viskosität der Produkte sehr hoch. Alkylmonoglycoside sind erst oberhalb von 120°C fließ-, rühr- und pumpfähig, während Alkyloligoglycoside selbst bei noch höheren Temperaturen Festkörper sind.

Andererseits sind die meisten Alkylglycoside thermisch nur begrenzt belastbar; die Zersetzungsreaktion setzt bereits bei Temperaturen ab 130°C merklich ein, was zu unerwünschter Dunkelfärbung der Produkte und Bildung von Zuckeralkoholen führt. Bei Verwendung von Dünnschichtverdampfern für die Destillation machen sich derartige Viskositätsprobleme besonders bemerkbar: Hochviskose Bestandteile der ungereinigten Reaktionsmischung backen im Dünnschichtverdampfer fest. Dabei werden in kurzer Zeit dunkel gefärbte Abbauprodukte

oder sogenannte "Zuckerkohle" gebildet, die zu einer deutlichen Qualitätsverschlechterung der erwünschten Produkte führt. Das in der US-PS 4 393 203 beanspruchte Reinigungsverfahren für Alkylglycoside ist aus diesen Gründen nur begrenzt und nur bei niederen Qualitätsansprüchen an die erhaltenen Produkte anwendbar.

In der EP-A 80 108 285.7 bzw. der DE-OS 30 01 064 wird ein Verfahren zur Reinigung von Alkylglycosiden mit 8 bis 16 C-Atomen im Alkylrest durch destillative Abtrennung nicht umgesetzter Fettalkohole offenbart, das darin besteht, zumindest den letzten Rest nicht umgesetzten Fettalkohols aus den Produktmischungen destillativ in Gegenwart von Glycolen abzutrennen, deren Siedepunkte die der abzutrennenden Alkohole um höchstens 10°C überschreiten und um höchstens 30°C unterschreiten. Durch den Glycol-Zusatz wird bewirkt, daß Reste von Fettalkoholen aus dem Produktgemisch ausgeschleppt werden können und außerdem für die Dauer des Destillationsvorgangs die Alkylglycoside in verdünnter und damit niedrigviskoser Form vorliegen. Nachteilig an diesem Verfahren ist vom Standpunkt technischer Herstellungprozesse, daß wiederum ein Schlepp- bzw. Lösungsmittel, nämlich ein Glycol oder ein Gemisch mehrerer Glycole, der Reaktionsmischung zugesetzt werden muß und damit ein weiterer Schritt der Wiedergewinnung und des Recyclings dieses "Hilfsstoffes" erforderlich wird.

Aufgabe der vorliegenden Erfindung war, die Zugabe gerader solcher Hilfsstoffe in der Reinigung von Alkylglycosiden zu vermeiden, da ihre Entfernung, Reinigung und Wiedereinschleusung in den Herstellungsprozeß zusätzliche und in der Regel aufwendige Verfahrensmaßnahmen erfordert. Es sollten vielmehr ohne zusätzliche Schritte der Trennung und Reinigung der Produkte von Fettalkohol-Resten völlig befreite Alkylglycoside zugänglich gemacht werden, die oberflächenaktive Eigenschaften zeigen und unmittelbar für die Verwendung in Kosmetika bzw. in Wasch-, Spül- und Reinigungsmitteln geeignet sind.

Überraschend wurde gefunden, daß Zusätze bestimmter polarer organischer Verbindungen zu Reaktionsmischungen, die Alkylglycoside und noch nicht umgesetzte Fettalkohol-Rückstände enthalten, nicht nur zu einer deutlichen Viskositätserniedrigung und damit besseren Handhabbarkeit der Reaktionsmischungen führen, sondern auch eine Schmelzpunkterniedrigung in den Alkylglycosiden mit sich bringen, die eine geringere thermische Belastung bei der destillativen Reinigung derartiger Reaktionslösungen zur Folge hat. Es entstehen dadurch bei Destillationstemperaturen, die weit unterhalb des thermischen Zersetzungspunktes der Alkylglycoside liegen, Fettalkohol-freie Reaktionsmischungen, die aufgrund ihrer niedrigen Viskosität technisch leicht zu handhaben sind und sich für den Einsatz in den genannten Bereichen bestens eignen, da auch eine Verbesserung der Wasserlöslichkeit der Produkte bewirkt wird.

Die Erfindung betrifft daher ein Verfahren zur Trennung von Alkylglycosiden und Fettalkoholen durch Destillation beide Komponenten enthaltender Reaktionsmischungen, das dadurch gekennzeichnet ist, daß man den Mischungen ein oder mehrere viskositätssenkende Mittel zusetzt, die

(a) thermostabil und farbstabil im Temperaturbereich bis 160°C und

..

(b) mit den Alkylglycosiden in der Reaktionsmischung kompatibel sind,

(c) bei 760 mbar einen Siedepunkt von 50°C oder mehr über dem Siedepunkt des in der Reaktionsmischung enthaltenen Fettalkohols aufweisen und

(d) bei Zusatz zu den Reaktionsmischungen im Temperaturbereich bis 160°C eine Viskosität von höchstens 10 000 mPas einstellen,

und die Fettalkohole aus den so modifizierten, niedrigviskosen Mischungen in an sich bekannten Vorrichtungen zur Destillation bei Temperaturen von mindestens 20°C unter dem thermischen Zersetzungspunkt der Alkylglycoside und Drücken von 1 bis $10^{-3}$ bar durch Destillation quantitativ abtrennt.

Die Erfindung betrifft außerdem Zusammensetzungen, die Alkylglycoside in einer Menge von 90 bis 50 Gew.-% und ein oder mehrere viskositätssenkende Mittel, die thermostabil und farbstabil im Temperaturbereich bis 160°C und mit den Alkylglycosiden in der Reaktionsmischung kompatibel sind, einen Siedepunkt von wenigstens 50°C über dem Siedepunkt des in der Reaktionsmischung vorhandenen Fettalkohols aufweisen und bei Zusatz zu den Reaktionsmischungen im Temperaturbereich bis 160°C eine Viskosität von höchstens 10 000 mPa . s einstellen, in einer Menge von 10 bis 50 Gew.-% enthalten.

Die Erfindung betrifft außerdem die Verwendung der genannten Zusammensetzungen zur Herstellung von Kosmetika sowie Wasch-, Spül- und Reinigungsmitteln für Haushalts- und Industriezwecke.

Im Zusammenhang mit dem erfindungsgemäßen Verfahren spielt die Viskosität der den an sich bekannten Vorrichtungen zur Destillation zugeführten Reaktionsmischungen eine wichtige Rolle. Als "pumpfähig" bzw. "pumpbar" werden im folgenden Lösungen bzw. flüssige Reaktionsgemische angesprochen, deren Viskosität nicht mehr als 10 000 mPa . s bei der jeweiligen Verfahrenstemperatur beträgt.

Als Zusätze zur Senkung der Viskosität von Alkylglycoside und Fettalkohole enthaltenden Reaktionsmischungen werden gemäß dem vorliegenden Verfahren Verbindungen zugesetzt, die thermostabil und farbstabil in dem für die Destillation in Frage kommenden Temperaturbereich bis 160°C und mit den Alkylglycosiden kompatibel, d.h. mischbar bzw. verträglich sind. Die Verbindungen müssen außerdem bei 760 mbar einen Siedepunkt von 50°C oder mehr über dem Siedepunkt des in der Reaktionsmischung enthaltenen Fettalkohols aufweisen und bei Zusatz zu den Reaktionsmischungen aus Alkylglycosiden und Fettalkoholen im Temperaturbereich bis 160°C eine Viskosität von höchstens 10 000 mPa . s einstellen. Mit Vorteil werden solche Verbindungen als viskositätssenkende Mittel verwendet, die die anwendungstechnisch angestrebte Wirkung der in den Reaktionsmischungen enthaltenen Alkylglycoside verbessern, indem sie beispielsweise eine die Wirkung der Alkylglycoside unterstützende anwendungstechnische Wirksamkeit als Detergenzien, Netzmittel, Emulgatoren, Antischaummittel oder Waschkraftverstärker aufweisen. Dabei kann aus der genannten Gruppe viskositätssenkender Mittel entweder eine Verbindung allein oder auch eine Mischung mehrerer derartiger viskositätssenkender Verbindungen mit obigen Eigenschaften zugesetzt werden.

Vorteilhafterweise setzt man den Reaktionsmischungen aus Alkylglycosiden und Fettalkoholen ein oder mehrere

viskositätssenkende Mittel zu, die aus den nachfolgenden Verbindungsgruppen ausgewählt sind:

(a) vicinale Diole und vicinale Hydroxyamine, abgeleitet aus den Epoxiden von Olefinen, ungesättigten Fettsäuren oder ungesättigten Fettalkoholen mit 8 bis 22 C-Atomen in der Kohlenstoffkette;

(b) Anlagerungsprodukte von Ethylenoxid und/oder Propylenoxid und/oder Butylenoxid an Fettderivate der allgemeinen Formel

$$Y\text{-}R^1\text{-}X,$$

in der

X für COOH, OH, $NR^2H$ oder $CONH_2$,

Y für H, OH oder $CH(OH)R^3$,

$R^1$ für unverzweigte oder verzweigte Alkylen- oder Alkenylenreste mit 8 bis 22 C-Atomen,

$R^2$ für H oder unverzweigte oder verzweigte Alkylreste mit 1 bis 8 C-Atomen oder für $(CH_2)_mNH_2$ mit m = 2 oder 3 und

$R^3$ für H oder unverzweigte oder verzweigte Alkylreste mit 1 bis 6 C-Atomen stehen,

(c) Anlagerungsprodukte von Ethylenoxid und/oder Propylenoxid an lineare oder cyclische Hydroxyverbindungen mit 1 bis 6 Hydroxygruppen und 1 bis 6 C-Atomen und an Polyglycerin und

(d) Etherderivate von Verbindungen der allgemeinen Formel

$$A\text{-}O\text{-}R^4,$$

in der

A ein Rest ist, der aus Verbindungen der oben genannten Gruppen (a), (b) oder (c) nach Abzug eines Wasserstoffatoms entsteht, und

$R^4$ für lineare oder verzweigte Alkyl- oder Alkenylreste mit 1 bis 18 C-Atomen, $CH_2OR^5$, $NR^7_2$ oder $R^6COOH$ steht, wobei $R^5$ einen linearen oder verzweigten Alkylrest mit 1 bis 18 C-Atomen, $R^6$ Methylen-, Ethylen- oder Trimethylenreste und $R^7$ Wasserstoff, einen Alkylrest mit 1 bis 4 C-Atomen oder einen Hydroxylkalenrest mit 1 bis 4 C-Atomen in der Alkylengruppe bedeuten können. Auch Fettalkyletheramine und insbesondere deren Ethoxylate sind geeignet.

In besonderen, gemäß der vorliegenden Erfindung bevorzugten Ausführungsformen werden in dem Verfahren zur Trennung von Alkylglycosiden und Fettalkoholen Fettalkoholethoxylate verwendet, die 12 bis 18 C-Atome im Alkylrest des Alkohols aufweisen und einen Gehalt von 3 bis 10 Mol Ethylenoxid pro Mol Fettalkohol haben. Bevorzugt werden Fettalkoholethoxylate mit 12 bis 14 C-Atomen im Alkylrest des Alkohols und einem Gehalt an 5 Mol Ethylenoxid pro Mol Fettalkohol. Dabei können die genannten Fettalkoholethoxylate einzeln oder in Mischungen miteinander zugesetzt werden. Beispiele von erfindungsgemäß verwendbaren Fettalkoholethoxylaten sind demnach Verbindungen der allgemeinen Formel

$$C_mH_{2m+1}O(CH_2CH_2O)_nH$$

in der

m für ganze Zahlen im Bereich von 12 bis 18, bevorzugt von 12 bis 14, und

n für ganze Zahlen im Bereich von 3 bis 10, bevorzugt für 5,

stehen, wobei die Alkylgruppe anstelle einzelner H-Atome Hydroxylsubstituenten enthalten kann.

Mit Vorteil werden Fettalkoholethoxylate aus der Gruppe der Addukte von Kokosalkohol an 5 Mol Ethylenoxid, Kokosalkohol an 3 Mol Ethylenoxid, Talgalkohol an 5 Mol Ethylenoxid und Oxoalkohol an 7 Mol Ethylenoxid verwendet. Dabei ist hier, wie auch im folgenden, unter "Kokosalkohol" ein Gemisch von Fettalkoholen synthetischer oder nativer Herkunft mit 12 bis 18 C-Atomen, unter "Talgalkohol" ein Gemisch von Fettalkoholen synthetischer oder nativer Herkunft mit 16 bis 18 C-Atomen und unter "Oxoalkohol" ein Gemisch von Fettalkoholen aus der Oxo-Synthese mit 14 und 15 C-Atomen zu verstehen. Als spezielle Verbindung aus der Gruppe der Fettalkoholethoxylate der oben angegebenen allgemeinen Formel, in der in der Alkylgruppe anstelle einzelner H-Atome Hydroxylsubstituenten stehen können, ist ein Addukt von 5-Hydroxy-3-oxa-pentadecanol-1 an 10 Mol Ethylenoxid zu nennen.

In einer weiteren bevorzugten Ausführungsform werden anstelle der genannten Fettalkoholethoxylate erfindungsgemäß zur Senkung der Viskosität 1,2-Alkandiole zugesetzt, die 12 bis 18 C-Atome, bevorzugt 12 bis 14 C-Atome im Alkylrest aufweisen. Die genannten Alkandiole sind einzeln oder in Mischungen miteinander in beliebigem Verhältnis verwendbar.

Die genannten 1,2-Alkandiole haben die allgemeine Formel

$$HOCH_2-CH(OH)-C_pH_{2p+1}$$

in der
p eine ganze Zahl im Bereich von 10 bis 16, bevorzugt im Bereich von 10 bis 12 bedeutet. Beispielsweise lassen sich erfindungsgemäß 1.2-Dodecandiol, 1.2-Tridecandiol, 1.2-Tetradecandiol, 1.2-Pentadecandiol,

1.2-Hexadecandiol, 1.2-Heptadecandiol und/oder 1.2-Octadecandiol verwenden, wobei prizipiell die geradkettigen und verzweigtkettigen Isomeren der genannten 1.2-Diole verwendbar sind, aufgrund ihrer Verfügbarkeit aus nativen Quellen jedoch die geradkettigen Isomeren bevorzugt verwendet werden. Aus dieser Gruppe sind 1.2-Dodecandiol, 1.2-Tridecandiol und 1.2-Tetradecandiol bevorzugt verwendbar, wobei mit besonderem Vorteil 1.2-Dodecandiol verwendet wird.

Die genannten Fettalkoholethoxylate oder 1,2-Alkandiole werden den Reaktionsmischungen, die Alkylglycoside und abzutrennende Fettalkohole enthalten, alternativ in einer Menge von 10 bis 50 Gew.-% zugesetzt. Die bevorzugte Menge liegt im Bereich von 20 bis 50 Gew.-%.

In einer weiteren Ausführungsform des erfindungsgemäßen Verfahrens zur Reinigung von Alkylglycosiden können als viskositätssenkende Mittel auch Anlagerungsprodukte von Ethylenoxid und/oder Propylenoxid an Fettalkohole, Fettamine und Fettsäuren, die die oben angegebene allgemeine Formel

$$Y-R^1-X$$

mit den oben angegebenen Bedeutungen für X, Y und $R^1$ haben, sowie Etherderivate von Verbindungen der oben angegebenen allgemeinen Formel

$$A-O-R^4$$

mit den oben angegebenen Bedeutungen für A und $R^4$ verwendet werden. Als solche Verbindungen kommen beispielsweise Anlagerungsprodukte von Kokosamin an 4 Mol Ethylenoxid, Kokosamin an 12 Mol Ethylenoxid, Anlagerungsprodukte von Kokosalkohol an 5 Mol Ethylenoxid und 4 Mol Propylenoxid oder an 4 Mol Ethylenoxid und 5 Mol Propylenoxid, Anlagerungsprodukte von technischer Ölsäure an 5,5 Mol Ethylenoxid, sowie 10-(Diethanolamino)octadecanol-1, Etherderivate der oben angegebenen allgemeinen Formel

$$A\text{-}O\text{-}R^4$$

in der

A ein Rest ist, der aus einem Addukt von Kokosalkohol an 9 Mol Ethylenoxid nach Abzug eines Wasserstoffatoms entsteht und

$R^4$ eine Alkylgruppe mit 1 bis 4 C-Atomen, bevorzugt mit 4 C-Atomen ist,

sowie außerdem Etherderivate derselben allgemeinen Formel, in der

A für einen Rest steht, der aus einem Addukt von Kokosalkohol an 1 bis 10 Mol, bevorzugt 4 bis 5 Mol, Ethylenoxid entsteht und

$R^4$ für einen Aminorest der Formel $NR^7_2$ mit der obigen Bedeutung für $R^7$ steht.

Die die genannten Zusätze enthaltenden Reaktionsmischungen werden in an sich bekannten Vorrichtungen zur Destillation erwärmt. Dabei handelt es sich beispielsweise um übliche, für die Sumpfphasendestillation oder für andere diskontinuierliche Destillationsverfahren geeignete Vorrichtungen oder auch um Dünnschichtverdampfer oder vergleichbare, dem Fachmann an sich bekannte Vorrichtungen für kontinuierliche Destillationsverfahren.

Die in dem erfindungsgemäßen Verfahren verwendeten Zusätze senken die Viskosität der Alkylglycoside und Fettalkohole enthaltenden Reaktionsmischungen deutlich. Gegenüber bekannten viskositätssenkenden Zusätzen haben sie den weiteren Vorteil, daß sie neben einer Viskositätserniedrigung überraschend auch eine Schmelzpunkterniedrigung der Alkylglycoside herbeiführen. Die Temperatur des Destillationsvorgangs zur Abtrennung überschüssiger Fettalkohole kann daher deutlich unter dem thermischen Zersetzungspunkt der Alkylglycoside liegen; er liegt in dem erfindungsgemäßen Verfahren mindestens 20°C unter dem thermischen Zersetzungspunkt der entsprechenden Verbindungen. Zudem wird die Wasserlöslichkeit der erhaltenen Alkylglycoside merklich erhöht.

Zur destillativen quantitativen Abtrennung der Fettalkohole aus der Reaktionsmischung wird im Regelfall bei Normaldruck gearbeitet. Um eine weitere Absenkung der Destillationstemperatur zu erreichen, kann die Destillation auch bei Unterdruck, beispielsweise im Bereich unterhalb von 1 bis hinunter zu $10^{-3}$ bar, durchgeführt werden. Dadurch lassen sich auch höhere Fettalkohole quantitativ aus den Reaktionsmischungen abtrennen.

Infolge der niedrigen Destillationstemperaturen färben sich die Reaktionsmischungen nicht infolge des Abbaus thermisch leicht zersetzbarer Alkylglycoside dunkel. Ein zusätzlicher, in bekannten Verfahren bei hohen Qualitätsanforderungen an das Produkt unumgänglicher Bleichschritt, in dem die Alkylglycoside enthaltenden Reaktionsmischungen mit Wasserstoffperoxidlösung gebleicht werden müssen, entfällt in dem erfindungsgemäßen Verfahren vollständig.

Es werden vielmehr Zusammensetzungen erhalten, die Alkylglycoside in einer Menge von 90 bis 50 Gew.-% und ein oder mehrere viskositätssenkende Mittel, die thermostabil und farbstabil im Temperaturbereich bis 160°C und mit den Alkylglycosiden in der Reaktionsmischung kompatibel sind, einen Siedepunkt von wenigstens 50°C über dem Siedepunkt des in der Reaktionsmischung vorhandenen Fettalkohols aufweisen und bei Zusatz zu den Reaktionsmischungen im Temperaturbereich bis 160°C eine Viskosität von höchstens 10 000 mPa . s einstellen, in einer Menge von 10 bis 50 Gew.-% enthalten. In den bevorzugten Verfahrensvarianten, die oben anhand der verwendeten Verbindungen ausführlich beschrieben wurden, werden Zusammensetzungen erhalten, die ein oder mehrere viskositätssenkende Mittel in einer Menge von 10 bis 50 Gew.-% enthalten, wobei die verwendeten Mittel die anwendungstechnisch angestrebten Wirkungen der Alkylglycoside verbessern, wie dies beispielsweise durch deren Wirksamkeit als Detergenzien, Netzmittel, Emulgatoren, Antischaummittel oder Waschkraftverstärker der Fall sein kann. Mit Vorteil werden Zusammensetzungen erhalten, die ein oder mehrere viskositätssenkende Mittel in einer Menge von 10 bis 50 Gew.-% enthalten, die ausgewählt sind aus der Klasse der nachfolgenden Verbindungen:

(a) vicinale Diole und vicinale Hydroxyamine, abgeleitet aus den Epoxiden von Olefinen, ungesättigten Fettsäuren oder ungesättigten Fettalkoholen mit 8 bis 22 C-Atomen in der Kohlenstoffkette;

(b) Anlagerungsprodukte von Ethylenoxid und/oder Propylenoxid und/oder Butylenoxid an Fettderivate der allgemeinen Formel

$$Y\text{-}R^1\text{-}X,$$

in der

X für COOH, OH, $NR^2H$ oder $CONH_2$,

Y für H, OH oder $CH(OH)R^3$,

$R^1$ für unverzweigte oder verzweigte Alkylen- oder Alkenylenreste mit 8 bis 22 C-Atomen,

$R^2$ für H oder unverzweigte oder verzweigte Alkylreste mit 1 bis 8 C-Atomen oder für $(CH_2)_mNH_2$ mit m = 2 oder 3 und

$R^3$ für H oder unverzweigte oder verzweigte Alkylreste mit 1 bis 6 C-Atomen stehen,

(c) Anlagerungsprodukte von Ethylenoxid und/oder Propylenoxid an lineare oder cyclische Hydroxyverbindungen mit 1 bis 6 Hydroxygruppen und 1 bis 6 C-Atomen und an Polyglycerin und

(d) Etherderivate von Verbindungen der allgemeinen Formel

$$A\text{-}O\text{-}R^4,$$

in der

A ein Rest ist, der aus Verbindungen der oben genannten Gruppen (a), (b) oder (c) nach Abzug eines Wasserstoffatoms entsteht, und

$R^4$ für lineare oder verzweigte Alkyl- oder Alkenylreste mit 1 bis 18 C-Atomen, $CH_2OR^5$, $NR^7_2$ oder $R^6COOH$ steht, wobei $R^5$ einen linearen oder verzweigten Alkylrest mit 1 bis 18 C-Atomen und $R^6$ Methylen-, Ethylen- oder Trimethylenreste und $R^7$ Wasserstoff, einen Alkylrest mit 1 bis 4 C-Atomen oder einen Hydroxyalkylenrest mit 1 bis 4 C-Atomen in der Alkylengruppe bedeuten können.

In besonders bevorzugten Ausführungsformen werden Zusammensetzungen erhalten, die als viskositätssenkende Mittel ein oder mehrere Fettalkoholethoxylate der angegebenen Strukturen oder alternativ ein oder mehrere

1,2-Alkandiole der angegebenen Strukturen in einer Menge von 10 bis 50 Gew.-% enthalten. Aufgrund ihrer besonders guten Anwendungsbreite werden bevorzugt Zusammensetzungen erhalten, die die gereinigten Alkylglycoside und Fettalkoholethoxylate bzw. 1,2-Alkandiole in einem Verhältnis von 80 : 20 bis 50 : 50 enthalten.

Für besondere Verwendungszwecke, beispielsweise in der Herstellung von Haarshampoos, ist es oftmals erwünscht, Alkylglycoside als 70 bis 80 %ige wässrige Lösungen zu konfektionieren. Dies läßt sich am einfachsten dadurch erreichen, daß die flüssige Alkylglycosid-Schmelze mit Wasser vermischt wird. Entsprechend dem vorliegenden Verfahren liegt infolge viskositätssenkender Zusätze der genannten Art, bevorzugt infolge der Zusätze von Fettalkoholethoxylaten oder 1,2-Alkandiolen, die auch die Wasserlöslichkeit der Produkte wesentlich verbessern, der Schmelzpunkt der Alkylglycosid-Mischungen unter 100°C. Dadurch ist die Einarbeitung der Mischungen in Wasser ohne Druck möglich, was in der Anwendungstechnik bevorzugt wird. Es werden dann als bevorzugte Zusammensetzungen solche erhalten, die Alkylglycoside in einer Menge von 75 bis 40 Gew.-%, Fettalkohole der oben genannten Struktur oder 1,2-Alkandiole in einer Menge von 5 bis 40 Gew.-% und Wasser in einer Menge von 20 bis 30 Gew.-% enthalten.

Derartige Zusammensetzungen sind vielseitig verwendbar. Sie weisen eine ausgezeichnete Mischbarkeit mit Wasser auf und können allein oder auch im Gemisch mit anderen Tensiden zur Herstellung von Kosmetika sowie in Wasch-, Spül- und Reinigungsmitteln für Haushalts- und Industriezwecke verwendet werden.

Die Erfindung wird durch die nachfolgenden Beispiele näher erläutert.

Beispiel 1

Eine Reaktionsmischung aus der Herstellung von Dodecyl-/Tetradecylmonoglucosid enthielt aus dem Herstellungsprozeß (Direktsynthese: Umsetzung von Dodecanol/Tetradecanol mit Glucose unter Katalyse mit Schwefelsäure zum Monoglucosid) noch a % des Fettalkohlgemisches. Das reine Monoglucosid ist bei 80°C fest und weist bei 130°C eine Viskosität von b Pa . s auf.

Der Reaktionsmischung wurde ein Fettalkoholethoxylat mit 12/14 C-Atomen im Alkylrest des Alkohols und einem durchschnittlichen Gehalt von 5 Mol Ethylenoxid pro Mol Fettalkohol zugesetzt. Bei den in der nachfolgenden Tabelle 1 angegebenen Mischungsverhältnissen wurde eine Verflüssigung bzw. Absenkung der Viskosität erreicht; die Werte sind ebenfalls der nachfolgenden Tabelle 1 zu entnehmen.

Tabelle 1

| | Alkylglycosid (Gew.-%) | Zusatz[a)] (Gew.-%) | Viskosität/Aggregatzustand bei 80°C (Pa.s) | Viskosität/Aggregatzustand bei 130°C (Pa.s) |
|---|---|---|---|---|
| a | 100 | 0 | —/fest | 3695 /flüssig |
| b | 80 | 20 | 1420 /rührfähige Paste | 91 /flüssig |
| c | 50 | 50 | 60 /flüssig | 12 /flüssig |

a) Anlagerungs-Produkt von Dodecanol/Tetradecanol an 5 EO

Beispiel 2

In gleicher Weise wie in Beispiel 1 wurden Reaktionsmischungen aus der Herstellung des Dodecyl-/Tetradecylmonoglucosids mit einer Mischung von 1,2-Dodecandiol und 1,2-Tetradecandiol in den in Tabelle 2 angegebenen Verhältnissen versetzt. Die Werte für die Viskosität der erhaltenen Zusammensetzungen sowie deren Aggregatzustand sind ebenfalls Tabelle 2 zu entnehmen.

Tabelle 2

| | Alkylglycosid (Gew.-%) | Zusatz[b)] (Gew.-%) | Viskosität/Aggregatzustand bei 80°C (Pa.s) | 130°C (Pa.s) |
|---|---|---|---|---|
| a | 100 | 0 | ——/fest | 3695 /flüssig |
| b | 80 | 20 | 4045 /flüssig | 139 /noch pumpbar |
| c | 50 | 50 | 199 /flüssig | 20 /flüssig |

b) 1,2-Dodecandiol/1,2-Tetradecandiol D7038

Beispiel 3

Eine Reaktionsmischung aus der Direktsynthese von Dodecyl-/Tetradecyloligoglucosid (durchschnittlicher Grad der Glucosidierung: 2,2) aus dem entsprechenden Fettalkohol-Gemisch und Glucose in Gegenwart von Schwefelsäure als Katalysator, das noch a % des Fettalkohols aus der Synthese enthielt, wurde in den in Tabelle 3 angegebenen Mengen mit einem Anlagerungsprodukt von Dodecanol/Tetradecanol an 5 EO in den in Tabelle 3 angegebenen Mengen versetzt. Die Reaktionsmischung

ohne viskositätssenkenden Zusatz wies bei 80°C eine feste und bei 130°C eine zähflüssige Konsistenz auf; bei Zugabe der in Tabelle 3 angegebenen Mengen des Fettalkoholethoxylats wurde eine deutliche Erniedrigung der Viskosität beobachtet.

Tabelle 3

| | Alkylglycosid (Gew.-%) | Zusatz[a] (Gew.-%) | Viskosität/Aggregatzustand bei 80°C (Pa.s) | Viskosität/Aggregatzustand bei 130°C (Pa.s) |
|---|---|---|---|---|
| a | 100 | 0 | ——/fest | 24650 /zähflüssig |
| b | 80 | 20 | 47200 /noch pumpbar | 1130 /flüssig |
| c | 50 | 50 | 181 /flüssig | 46 /flüssig |

a) Anlagerungsprodukt von Dodecanol/Tetradecanol an 5 EO

Beispiel 4

Die in Beispiel 3 beschriebene Reaktionsmischung wurde in dem in Tabelle 4 angegebenen Verhältnis mit einer Mischung aus 1,2-Dodecandiol und 1,2-Tetradecandiol versetzt. Auch durch Zusatz der Diolmischung konnte eine deutliche Verringerung der Viskosität erreicht werden. Die Meßwerte sind im einzelnen Tabelle 4 zu entnehmen.

Tabelle 4

| | Alkylglycosid (Gew.-%) | Zusatz[b)] (Gew.-%) | Viskosität/Aggregatzustand bei 80°C (Pa.s) | Viskosität/Aggregatzustand bei 130°C (Pa.s) |
|---|---|---|---|---|
| a | 100 | 0 | —— /fest | 24650 /zähflüssig |
| b | 80 | 20 | —— /noch pumpbar | 1835 /flüssig |
| c | 50 | 50 | 610 /flüssig | 68 /flüssig |

b) 1,2-Dodecandiol/1,2-Tetradecandiol

Beispiel 5

Die in den Beispielen 1 bis 4 beschriebenen Reaktionsmischungen wurden einer kontinuierlichen Destillation an einem Dünnschichtverdampfer unterworfen. Der Dünnschichtverdampfer hatte die nachfolgenden apparativen Parameter:

Fläche des Heizmantels: 500 cm²;
Drehzahl : 285 $min^{-1}$;
Umfangsgeschwindigkeit: 76 cm . $s^{-1}$.

Die beschriebenen Reaktionsmischungen wurden über einen mit Wasserdampf auf ca. 60 bis 80°C geheizten Zulauf-Tropftrichter zugegeben. Der Kühlfinger des Dünnschichtverdampfers wurde je nach Art des Fettalkohols, d.h. in Abhängigkeit von dessen Schmelzpunkt, mit kaltem oder warmem Wasser betrieben. Destillat bzw. Rückstand wurden in getrennten Vorlagekolben aufgefangen. Gegebenenfalls wurde der Auslaufstutzen des Dünnschichtverdampfers zusätzlich mit Hilfe eines Wärmestrahlers beheizt, um nicht glatt ablaufende Substanzanteile aufzuschmelzen.

Die Betriebsparameter sowie der Restgehalt an Fettalkohol in der Mischung nach dem ersten Destillationsdurchlauf sind in Tabelle 5 angegeben.

Bei der kontinuierlichen Destillation der in den Beispielen 1 bis 4 beschriebenen Reaktionsmischungen wurde gefunden, daß die keinen viskositätssenkenden Zusatz enthaltenden Chargen nicht nur schon bei Raumtemperatur wesentlich schwerer handhabbar waren, sondern auch bei erhöhter Temperatur nur unter Schwierigkeiten destilliert werden konnten. Immer wieder wurde ein Festbacken von Alkylglucosidanteilen im Auslaufstutzen des Dünnschichtverdampfers beobachtet. Außerdem resultierte ein sehr hoher Restgehalt an Fettalkohol nach einem Destillationszyklus.

Im Gegensatz dazu konnten die viskositätssenkende Zusätze enthaltenden Reaktionsmischungen (vgl. Beispiele 1 bis 4) ohne Schwierigkeiten im Dünnschichtverdampfer destilliert werden. Ein Festkleben oder Stocken von Alkylglucosid-Anteilen war nicht zu beobachten. Die Reaktionsmischungen liefen in einem zusammenhängenden Film ab. Der Restgehalt an Fettalkohol konnte schon im ersten Destillationszyklus auf einen sehr niedrigen Wert abgesenkt werden.

Vergleichsbeispiel 1

Eine Reaktionsmischung aus der Direktsynthese von Dodecylmonoglucosid, die noch a Gew.-% nicht umgesetzten Dodecanols enthielt, wurde ohne Zusatz eines Fettalkoholethoxylats oder 1,2-Alkandiols im Dünnschichtverdampfer destilliert. Die Verfahrensparameter sind in Tabelle 5 angegeben. Bei einem Durchsatz von 120 ml der Reaktionsmischung pro Minute wurde beobachtet, daß schlecht lösliche Anteile der Reaktionsmischung im Dünnschichtverdampfer festklebten und erst nach Herausschmelzen abliefen. Bei einem Durchsatz von 120 ml der Reaktionsmischung pro Minute enthielt das Destillat nach einem Destillationszyklus noch einen Restgehalt an Dodecanol von 11 %.

Vergleichsbeispiel 2

Eine Reaktionsmischung aus der Direktsynthese von Dodecylmonoglucosid, die noch b Gew.-% noch nicht umgesetzten Dodecanols enthielt, wurde ohne Zusatz eines viskositätssenkenden Mittels einer Dünnschichtdestillation unter den in Tabelle 5 angegebenen Verfahrensparametern unterworfen. Auch in diesem Fall wurde ein Festkleben nicht geschmolzener Bestandteile der Reaktionsmischung beobachtet; diese mußten durch Erwärmen aus dem Dünnschichtverdampfer herausgeschmolzen werden. Nach einem Destillationszyklus betrug der Restgehalt an Dodecanol weniger als 1 %.

Tabelle 5

| Mischung aus Bsp. | Temp. des Heizmantels (°C) | Druck (mbar) | Durchsatz (ml/min) | Restgehalt R-OH (Gew.-%) |
|---|---|---|---|---|
| 1 b | 100 | 10 - 2 | 170 | 5,8 |
| 2 | | | | |
| 3 | | | | |
| 4 | | | | |
| V1 | 100 | 10 - 2 | 120 | 11,0 |
| V2 | 150 | 10 - 1 | 180 | <1 |

## Patentansprüche

1. Verfahren zur Trennung von Alkylglycosiden und Fettalkoholen durch Destillation beide Komponenten enthaltender Reaktionsmischungen, dadurch gekennzeichnet, daß man den Mischungen ein oder mehrere viskositätssenkende Mittel zusetzt, die
(a) thermostabil und farbstabil im Temperaturbereich bis 160°C und
(b) mit den Alkylglycosiden in der Reaktionsmischung kompatibel sind,
(c) bei 760 mbar einen Siedepunkt von 50°C oder mehr über dem Siedepunkt des in der Reaktionsmischung enthaltenen Fettalkohols aufweisen und
(d) bei Zusatz zu den Reaktionsmischungen im Temperaturbereich bis 160°C eine Viskosität von höchstens 10 000 mPas einstellen,
und die Fettalkohole aus den so modifizierten, niedrigviskosen Mischungen in an sich bekannten Vorrichtungen zur Destillation bei Temperaturen von mindestens 20°C unter dem thermischen Zersetzungspunkt der Alkylglycoside und Drücken von 1 bis $10^{-3}$ bar durch Destillation quantitativ abtrennt.

2. Verfahren nach Anspruch 1, dadurch gekennzeichnet, daß man den Mischungen ein oder mehrere viskositätssenkende Mittel zusetzt, die die anwendungstechnisch angestrebte Wirkung der in den Reaktionsmischungen enthaltenen Alkylglycoside verbessern.

3. Verfahren nach Ansprüchen 1 und 2, dadurch gekennzeichnet, daß man den Mischungen ein oder mehrere viskositätssenkende Mittel zusetzt, die anwendungstechnisch wirksam sind als Detergens, Netzmittel, Emulgator, Antischaummittel oder Waschkraftverstärker.

4. Verfahren nach Ansprüchen 1 bis 3, dadurch gekennzeichnet, daß man den Mischungen ein oder mehrere viskositätssenkende Mittel zusetzt, die ausgewählt sind aus den nachfolgenden Verbindungsgruppen:

(a) vicinale Diole und vicinale Hydroxyamine, abgeleitet aus den Epoxiden von Olefinen, ungesättigten Fettsäuren oder ungesättigten Fettalkoholen mit 8 bis 22 C-Atomen in der Kohlenstoffkette;

(b) Anlagerungsprodukte von Ethylenoxid und/oder Propylenoxid und/oder Butylenoxid an Fettderivate der allgemeinen Formel

$$Y\text{-}R^1\text{-}X,$$

in der

X für COOH, OH, $NR^2H$ oder $CONH_2$,

Y für H, OH oder $CH(OH)R^3$,

$R^1$ für unverzweigte oder verzweigte Alkylen- oder Alkenylenreste mit 8 bis 22 C-Atomen,

$R^2$ für H oder unverzweigte oder verzweigte Alkylreste mit 1 bis 8 C-Atomen oder für $(CH_2)_mNH_2$ mit m = 2 oder 3 und

$R^3$ für H oder unverzweigte oder verzweigte Alkylreste mit 1 bis 6 C-Atomen stehen,

(c) Anlagerungsprodukte von Ethylenoxid und/oder Propylenoxid an lineare oder cyclische Hydroxyverbindungen mit 1 bis 6 Hydroxygruppen und 1 bis 6 C-Atomen und an Polyglycerin und

(d) Etherderivate von Verbindungen der allgemeinen Formel

$$A\text{-}O\text{-}R^4,$$

in der
A ein Rest ist, der aus Verbindungen der oben genannten Gruppen (a), (b) oder (c) nach Abzug eines Wasserstoffatoms entsteht, und
$R^4$ für lineare oder verzweigte Alkyl- oder Alkenylreste mit 1 bis 18 C-Atomen, $CH_2OR^5$, $NR^7_2$ oder $R^6COOH$ steht, wobei $R^5$ einen linearen oder verzweigten Alkylrest mit 1 bis 18 C-Atomen, $R^6$ Methylen-, Ethylen- oder Trimethylenreste und $R^7$ Wasserstoff, einen Alkylrest mit 1 bis 4 C-Atomen oder einen Hydroxylkalenrest mit 1 bis 4 C-Atomen in der Alkylengruppe bedeuten können.

5. Verfahren nach Ansprüchen 1 bis 4, dadurch gekennzeichnet, daß man den Mischungen ein oder mehrere viskositätssenkende Mittel aus der Gruppe
(a) Fettalkoholethoxylate mit 12 bis 18 C-Atomen im Alkylrest des Alkohols und einem Gehalt von 3 bis 10 Mol Ethylenoxid pro Mol Fettalkohol oder aus der Gruppe
(b) 1,2-Alkandiole mit 12 bis 18 C-Atomen im Alkylrest zusetzt.

6. Verfahren nach Anspruch 5, dadurch gekennzeichnet, daß man den Mischungen Fettalkoholethoxylate oder 1,2-Alkandiole in einer Menge von 10 bis 50 Gew.-% zusetzt.

7. Verfahren nach Ansprüchen 1 bis 6, dadurch gekennzeichnet, daß man Fettalkoholethoxylate mit 12 bis 14 C-Atomen im Alkylrest des Alkohols und einem Gehalt an 5 Mol Ethylenoxid pro Mol Fettalkohol zusetzt.

8. Verfahren nach Ansprüchen 1 bis 7, dadurch gekennzeichnet, daß man 1,2-Alkandiole mit 12 bis 14 C-Atomen einzeln oder in Mischungen miteinander zusetzt.

9. Verfahren nach Ansprüchen 1 bis 8, dadurch gekennzeichnet, daß man Fettalkoholethoxylate oder 1,2-Alkandiole in einer Menge von 20 bis 50 Gew.-% zusetzt.

10. Zusammensetzungen enthaltend
(a) Alkylglycoside in einer Menge von 90 bis 50 Gew.-% und
(b) ein oder mehrere viskositätssenkende Mittel, die thermostabil und farbstabil im Temperaturbereich bis 160°C und mit den Alkylglycosiden in der Reaktionsmischung kompatibel sind, einen Siedepunkt von wenigstens 50°C über dem Siedepunkt des in der Reaktionsmischung vorhandenen Fettalkohols aufweisen und bei Zusatz zu den Reaktionsmischungen im Temperaturbereich bis 160°C eine Viskosität von höchstens 10 000 mPas einstellen, in einer Menge von 10 bis 50 Gew.-%.

11. Zusammensetzungen nach Anspruch 10, dadurch gekennzeichnet, daß sie ein oder mehrere viskositätssenkende Mittel in einer Menge von 10 bis 50 Gew.-% enthalten, die die anwendungstechnisch angestrebten Wirkungen der Alkylglycoside verbessern.

12. Zusammensetzungen nach Ansprüchen 10 und 11, dadurch gekennzeichnet, daß sie ein oder mehrere viskositätssenkende Mittel in einer Menge von 10 bis 50 Gew.-% enthalten, die anwendungstechnisch wirksam sind als Detergentien, Netzmittel, Emulgatoren, Antischaummittel oder Waschkraftverstärker.

13. Zusammensetzungen nach Ansprüchen 10 bis 12, dadurch gekennzeichnet, daß sie ein oder mehrere viskositätssenkende Mittel in einer Menge von 10 bis 50 Gew.-% enthalten, die ausgewählt sind aus der Klasse folgender Verbindungen:

(a) vicinale Diole und vicinale Hydroxyamine, abgeleitet aus den Epoxiden von Olefinen, ungesättigten Fettsäuren oder ungesättigten Fettalkoholen mit 8 bis 22 C-Atomen in der Kohlenstoffkette;

(b) Anlagerungsprodukte von Ethylenoxid und/oder Propylenoxid und/oder Butylenoxid an Fettderivate der allgemeinen Formel

$$Y\text{-}R^1\text{-}X,$$

in der

X für COOH, OH, $NR^2H$ oder $CONH_2$,

Y für H, OH oder $CH(OH)R^3$,

$R^1$ für unverzweigte oder verzweigte Alkylen- oder Alkenylenreste mit 8 bis 22 C-Atomen,

$R^2$ für H oder unverzweigte oder verzweigte Alkylreste mit 1 bis 8 C-Atomen oder für $(CH_2)_mNH_2$ mit m = 2 oder 3 und

$R^3$ für H oder unverzweigte oder verzweigte Alkylreste mit 1 bis 6 C-Atomen stehen,

(c) Anlagerungsprodukte von Ethylenoxid und/oder Propylenoxid an lineare oder cyclische Hydroxyverbindungen mit 1 bis 6 Hydroxygruppen und 1 bis 6 C-Atomen und an Polyglycerin und

(d) Etherderivate von Verbindungen der allgemeinen Formel

$$A\text{-}O\text{-}R^4,$$

in der

A ein Rest ist, der aus Verbindungen der oben genannten Gruppen (a), (b) oder (c) nach Abzug

eines Wasserstoffatoms entsteht, und

$R^4$ für lineare oder verzweigte Alkyl- oder Alkenylreste mit 1 bis 18 C-Atomen, $CH_2OR^5$, $NR^7_2$ oder $R^6COOH$ steht, wobei $R^5$ einen linearen oder verzweigten Alkylrest mit 1 bis 18 C-Atomen und $R^6$ Methylen-, Ethylen- oder Trimethylenreste und $R^7$ Wasserstoff, einen Alkylrest mit 1 bis 4 C-Atomen oder einen Hydroxyalkylenrest mit 1 bis 4 C-Atomen in der Alkylengruppe bedeuten können.

14. Zusammensetzungen nach Ansprüchen 10 bis 13, dadurch gekennzeichnet, daß sie als viskositätssenkende Mittel ein oder mehrere Fettalkoholethoxylate mit 12 bis 18 C-Atomen im Alkylrest des Alkohols und einem Gehalt von 3 bis 10 mol Ethylenoxid pro mol Fettalkohol oder 1,2-Alkandiole mit 12 bis 18 C-Atomen im Alkylrest in einer Menge von 10 bis 50 Gew.-% enthalten.

15. Zusammensetzungen nach Ansprüchen 10 bis 14, dadurch gekennzeichnet, daß sie
(a) die gereinigten Alkylglycoside und
(b) Fettalkoholethoxylate oder 1,2-Alkandiole in einem Verhältnis der Komponente (a) : (b) von 80 : 20 bis 50 : 50 enthalten.

16. Zusammensetzungen nach Anspruch 10, enthaltend
(a) Alkylglycoside in einer Menge von 75 bis 40 Gew.-%,
(b) Fettalkoholethoxylate mit 12 bis 18 C-Atomen im Alkylrest des Alkohols und einem Gehalt von 3 bis 10 mol Ethylenoxid pro mol Fettalkohol oder 1,2-Alkandiole mit 12 bis 18 C-Atomen im Alkylrest in einer Menge von 5 bis 40 Gew.-% sowie
(c) Wasser in einer Menge von 20 bis 30 Gew.-%.

17. Verwendung der Zusammensetzungen nach Ansprüchen 10 bis 16 allein oder im Gemisch mit weiteren Tensiden zur Herstellung von Kosmetika und Wasch-, Spül- und Reinigungsmitteln für Haushalts- und Industriezwecke.